(19) Europäisches Patentamt
European Patent Office
Office européen des brevets

(11) Veröffentlichungsnummer: **0 022 971**
**B1**

(12) # EUROPÄISCHE PATENTSCHRIFT


(45) Veröffentlichungstag der Patentschrift: **16.03.83**

(21) Anmeldenummer: **80103847.2**

(22) Anmeldetag: **07.07.80**

(51) Int. Cl.³: **C 07 C 61/40**, C 07 C 69/743, C 07 C 51/363, C 07 C 67/343 // C07F9/40, C07F9/42


(54) **Verfahren zur Herstellung von 3-(2,2-Dichlor-vinyl)-2,2-dimethyl-cyclopropan-1-carbonsäure-derivaten.**


(30) Priorität: **11.03.80 DE 3009242**
**21.07.79 DE 2929725**

(43) Veröffentlichungstag der Anmeldung: **28.01.81 Patentblatt 81/4**

(45) Bekanntmachung des Hinweises auf die Patenterteilung: **16.03.83 Patentblatt 83/11**

(84) Benannte Vertragsstaaten: **BE CH DE FR GB IT LI NL**



(73) Patentinhaber: **BAYER AG, Zentralbereich Patente, Marken und Lizenzen, D-5090 Leverkusen 1, Bayerwerk (DE)**

(72) Erfinder: **Hoffmann, Hellmut, Dr., Tersteegenweg 17, D-5600 Wuppertal-1 (DE)**
Erfinder: **Maurer, Fritz, Dr., Roeberstrasse 8, D-5600 Wuppertal-1 (DE)**
Erfinder: **Priesnitz, Uwe, Dr., Heinrich-Heine-Strasse 42, D-4750 Unna-Massen (DE)**
Erfinder: **Riebel, Hans-Jochem, Dr., In der Beek 92, D-5600 Wuppertal-1 (DE)**



(56) Entgegenhaltungen:
**EP-A-0 000 345**
**EP-A-0 006 205**
**DE-A-1 807 091**
**DE-A-1 935 386**

**SYNTHESIS, Nr. 8, August 1975, P. SAVIGNAC et al.: „alpha-Chlorination and carbonyl olefination", Seite 535**
**CHIMIA, Band 28, Nr. 11, November 1974, Zürich, CH, H. ZIMMER et al.: „Syntheses with alpha-heterosubstituted phosphonate carbanions (III): diacetylenes and substituted vinyl chlorides", Seite 656**
**CHEMICAL ABSTRACTS, Band 92, Nr. 17, 28. April 1980, Zusammenfassung Nr. 146332f, Columbus, Ohio, US**
**„J. American Chem. Soc." 84(1962). S. 854 ff.**


**EP 0 022 971 B1**


Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 3-(2,2-Dichlorvinyl)-2,2-dimethylcyclopropan-1-carbonsäurederivaten

Die Erfindung betrifft ein neues Verfahren zur Herstellung von bekannten 3-(2,2-Dichlorvinyl)-2,2-dimethylcyclopropan-1-carbonsäurederivaten.

Es ist bekannt, dass man 3-(2,2-Dichlorvinyl)-2,2-dimethylcyclopropan-1-carbonsäureester erhält, wenn man 3-Formyl-2,2-dimethylcyclopropan-1-carbonsäureester mit Triphenylphosphin in Tetrachlorkohlenstoff umsetzt (vgl. DE-OS Nr. 2326077).

Nach dieser Synthesemethode erhält man die gewünschen Produkte jedoch nur in mässigen Ausbeuten.

Es ist weiter bekannt, dass man 1,1-Dichloralkene erhält, wenn man Lithiumsalze von Dichlormethanphosphonsäureestern mit Aldehyden oder Ketonen umsetzt (vgl. „Synthesis" *1975*, 535-536).

Die Herstellung der Lithiumsalze von Dichlormethanphosphonsäureestern ist jedoch relativ aufwendig: man erhält sie aus Chlormethanphosphonsäureestern durch Umsetzung mit Butyllithium und Tetrachlorkohlenstoff bei −75 °C, wobei sorgfältig getrocknete Lösungsmittel zu verwenden sind und eine Inertgasatmosphäre erforderlich ist.

Aus DE-OS Nr. 1807091 ist bekannt, dass bei der Umsetzung von 1-Methoxycarbonyläthyl-O,O-dimethylphosphonat mit 3,3-Dimethyl-2-(R)-formyl-1-(R)-cyclopropancarbonsäure in Gegenwart von Natriumamid und Tetrahydrofuran die entsprechende Methylmethoxycarbonylvinylverbindung entsteht. Bei der Bildung der Dihalogenvinylverbindungen war jedoch zu erwarten gewesen, dass, infolge des Entstehens einer Nebenreaktion bei der Methylenchlorid, die Ausbeute nur gering wird [vgl. „J. Am. Chem. Sa." 84 (1962), S. 854 ff.].

Aus EP-OS Nr. 345 war bekannt, dass bei der Umsetzung von Cyclopropanaldehyd mit Methanphosphonsäureestern in Gegenwart von Butyllithiumvinylcyclopropanderivate entstehen.

Der Einsatz von Butyllithium ist jedoch kostenaufwendig.

Es wurde nun gefunden, dass man 3-(2,2-Dichlorvinyl)-2,2-dimethylcyclopropan-1-carbonsäurederivate der Formel I

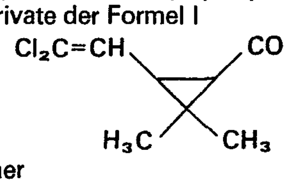

(I)

in welcher

R für Wasserstoff, ein Alkalimetall- oder Erdalkalimetalläquivalent, Alkyl oder für einen im Alkoholteil von Perythroiden üblichen Rest steht, erhält, wenn man 3-Formyl-2,2-dimethylcyclopropan-1-carbonsäurederivate der Formel II

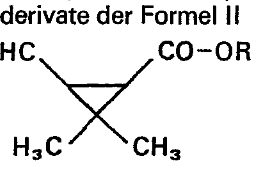

(II)

in welcher

R die oben angegebene Bedeutung hat, mit Dichlormethanderivaten der Formel III

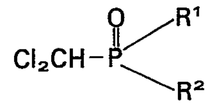

(III)

in welcher

$R^1$ und $R^2$ einzeln für Alkyl, Phenyl, Alkoxy, Phenoxy oder zusammen für Alkandiyl (Alkylen) oder Alkandioxy (Alkylendioxy) stehen,

gegebenfalls unter Verwendung eines Verdünnungsmittels vorlegt und mit einem Alkoholat bei Temperaturen zwischen −50 und +50 °C versetzt und anschliessend in üblicher Weise aufarbeitet.

Es ist als überraschend anzusehen, dass man nach dem erfindungsgemässen Verfahren 3-(2,2-Dichlorvinyl)-2,2-dimethylcyclopropan-1-carbonsäurederivate wesentlich einfacher und kostengünstiger in guten Ausbeuten erhält als man in Anbetracht des Standes der Technik erwarten konnte. Das Verfahren eignet sich ausserdem zur stereoselektiven Synthese der cis- bzw. trans-Verbindungen der Formel I, da bei der Einführung der Doppelbindung weitgehend keine Isomerisierung der entsprechenden Ausgangsverbindungen auftritt.

Verwendet man als Ausgangstoffe beispielsweise Dichlormethanphosphonsäuredimethylester und 3-Formyl-2,2-dimethylcyclopropan-1-carbonsäureisopropylester sowie als Base Kaliumisopropylat, so kann die Reaktion dieser Verbindungen durch folgendes Formelschema skizziert werden:

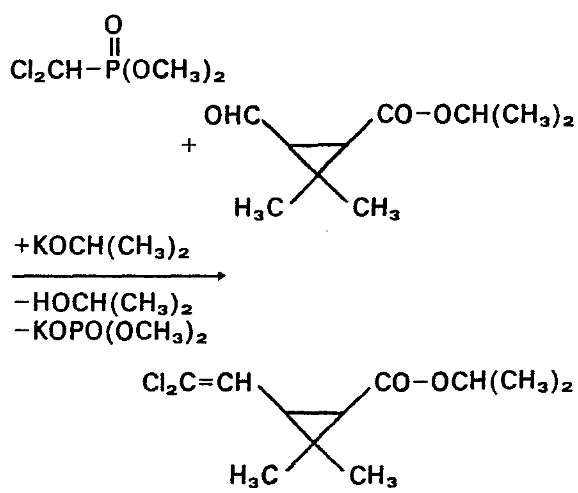


Die als Ausgangsstoffe zu verwendenden 3-Formyl-2,2-dimethylcyclopropan-1-carbonsäurederivate sind durch Formel II definiert. Vorzugsweise steht darin.

R für Wasserstoff, Natrium, Kalium oder für geradkettiges oder verzweigtes Alkyl mit 1 bis 4 Kohlenstoffatomen.

Als Beispiele seien 3-Formel-2,2-dimethylcyclopropan-1-carbonsäure, deren Natrium und Kaliumsalz sowie 3-Formyl-2,2-dimethylcyclo-

propan-1-carbonsäuremethylester, -äthylester, -n-propylester, -isopropylester, -n-butylester, -isobutylester, -sek.-butylester und tert.-butylester genannt.

Ausgangsverbindungen der Formel II sind bekannt (vgl. Nr. DE-OS 2615160; „Tetrahedron Lett." *1978*, 1847-1850; Nr. US-P 3679667).

Die weiter als Ausgangsverbindungen zu verwendenden Dichlomethanderivate sind durch Formel III definiert. Vorzugsweise stehen darin

$R^1$ und $R^2$ einzeln für Alkoxy mit 1 bis 4 Kohlenstoffatomen oder für Phenoxy oder beide Reste; $R^1$ und $R^2$ stehen zusammen für geradkettiges oder verzweigtes Alkandioxy (Alkylendioxy) mit 2 bis 5 Kohlenstoffatomen.

Als Beispiele seien Dichlormethanphosphonsäuredimethylester, -diäthylester und -diphenylester genannt.

Die Verbindungen der Formel III sind bekannt oder können nach an sich bekannten Verfahren hergestellt werden (vgl. „Synthesis" *1975*, 535-536; „Tetrahedron Letters" *1975*, 609-610; *ibid.* *1975*, 4409-4410).

Man erhält Dichlormethanphosphonsäureester der Formel III beispielsweise durch Umsetzung von Dichlormethanphosphonsäuredichlorid (vgl. GB-PS Nr. 707961) mit Natrium- oder Kaliumsalzen von Hydroxyverbindungen, wie z.B. mit Natrium- oder Kaliummethylat, -äthylat, -n- und -isopropylat, -n-, iso-, sek.- und tert.- butylat, gegebenenfalls in Gegenwart eines Verdünnungsmittels, wie z.B. Toluol, bei Temperaturen zwischen 0 und 50°C. Zur Reinigung der Produkte wird gegebenenfalls nach Filtration destilliert.

Das erfindungsgemässe Verfahren wird vorzugsweise unter Verwendung von Verdünnungsmitteln durchgeführt. Als solche kommen praktisch alle inerten organischen Lösungsmittel, insbesondere aprotisch polare Solvenzien in Frage. Hierzu gehören Äther, wie z.B. Glycoldimethyläther, Diglycoldimethyläther, Tetrahydrofuran und Dioxan, Carbonsäureamide, wie z.B. Dimethylformamid, Dimethylacetamid und N-Methylpyrrolidon, Sulfoxide und Sulfone, wie z.B. Dimethylsulfoxid und Tetramethylensulfon, Phosphorsäureamide, wie z.B. Hexamethylphosphorsäuretriamid, sowie Nitrile, wie z.B. Acetonitril und Propionitril.

Beim erfindungsgemässen Verfahren werden als Basen Alkoholate wie Alkalialkoholate, wie z.B. Natrium- und Kaliummethylat, Natrium- und Kaliumethylat, Natrium- und Kaliumisopropylat, Natrium- und Kalium-tert.-butylat, verwendet.

Die Reaktionstemperatur wird zwischen −50 und +50°C, vorzugsweise zwischen −30 und +30°C gehalten. Das erfindungsgemässe Verfahren wird im allgemeinen bei Normaldruck durchgeführt.

Je Mol 3-Formyl-2,2-dimethylcyclopropan-1-carbonsäurederivat der Formel II werden 1 bis 1,5 mol, vorzugsweise 1 bis 1,2 mol Dichlormethanderivat der Formel III und 1 bis 1,5 mol, vorzugsweise 1 bis 1,2 mol Base eingesetzt.

Die Ausgangsstoffe der Formeln II und III werden in einem der oben angegebenen Verdünnungsmittel bei Temperaturen zwischen −50 und +50°C bevorzugt zwischen −30 und +10°C, vorgelegt und mit der Lösung einer Base in einem der oben angegebenen Verdünnungsmittel tropfenweise versetzt. Dann lässt man das Reaktionsgemisch auf Raumtemperatur kommen und rührt einige Stunden nach.

Die Aufarbeitung erfolgt auf übliche Weise:

Man giesst das Reaktionsgemisch in Wasser und extrahiert mit einem mit Wasser nicht mischbaren Lösungsmittel, wie z.B. Methylenchlorid. Die Extrakte werden mit verdünnter Natronlauge und dann mit Wasser gewaschen, getrocknet und filtriert. Vom Filtrat wird das Lösungsmittel abgezogen und das als Rückstand verbleibende Produkt durch Vakuumdestillation gereinigt. Zur Charakterisierung dient der Siedepunkt.

Da die als Ausgangsverbindungen eingesetzten 3-Formyl-2,2-dimethylcyclopropan-1-carbonsäurederivate der Formel II wie auch die erfindungsgemäss herzustellenden 3-(2,2-Dichlorvinyl)-2,2-dimethylcyclopropan-1-carbonsäurederivate der Formel I jeweils asymmetrische Kohlenstoffatome aufweisen, können die Verbindungen der Formeln I und II in einer entsprechenden Anzahl von stereoisomeren Formen auftreten.

Das erfindungsgemässe Verfahren bezieht sich auf die stereoselektive Herstellung von Verbindungen der Formel I, die entweder in Form der einzelnen Stereoisomeren oder als Gemische von Stereoisomeren erhalten werden.

Die nach den erfindungsgemässen Verfahren herzustellenden 2,2-Dimethyl-3-(2,2-dichlorvinylcyclopropan-1-carbonsäurederivate können als Zwischenprodukte zur Herstellung von insektizid und akarizid wirksamen Pyrethoiden verwendet werden (vgl. DE-OS Nr. 2326077).

*Beispiel 1*

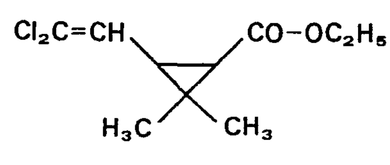

Zu einer Mischung von 48 g (0,22 mol) Dichlormethanphosphonsäurediäthylester und 34 g (0,2 mol) 3-Formyl-2,2-dimethylcyclopropan-1-carbonsäureäthylester in 200 ml Tetrahydrofuran wird bei −15°C eine Suspension von 24,6 g (0,22 mol) Kalium-tert.-butylat in 20 ml Dimethylformamid tropfenweise gegeben. Nach beendeter Zugabe wird das Reaktionsgemisch auf 20°C erwärmt und 2 h bei dieser Temperatur gerührt.

Dann wird das Reaktionsgemisch in 400 ml Wasser gegossen. Die wässerige Lösung wird zweimal mit je 200 ml Methylenchlorid extrahiert. Die vereinigten organischen Extrakte werden einmal mit 100 ml 5%iger Natronlauge und zweimal mit Wasser gewaschen, über Natriumsulfat getrocknet und dann eingedampft. Der Rückstand wird fraktioniert destilliert. Man erhält 42 g (88,6% der Theorie) 3-(2,2-Dichlorvinyl)-2,2-dimethylcyclopropan-1-carbonsäureäthylester in Form eines farblosen Öls vom Siedepunkt 80-95 °C/ 2 mbar.

*Beispiel 2*

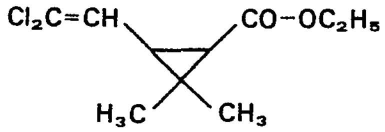

Die Umsetzung wird analog Beispiel 1 mit den gleichen Ausgangsverbindungen durchgeführt, wobei jedoch an Stelle von Kalium-tert.-butylat als Base Natriumäthylat (15 g) verwendet wird und die Reaktionstemperatur am Anfang bei +10 °C gehalten wird. Man erhält 24,5 g (51,6% der Theorie) 3-(2,2-Dichlorvinyl)-2,2-dimethylcyclopropan-1-carbonsäureäthylester in Form eines farbloses Öls vom Siedepunkt 80-95 °C/ 2 mbar.

*Beispiel 3*

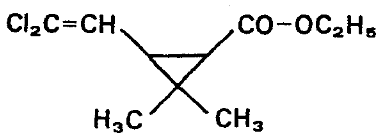

Die Umsetzung wird analog Beispiel 1 mit den gleichen Ausgangsverbindungen durchgeführt, wobei jedoch an Stelle einer Lösung von Kalium-tert.-butylat in Dimethylformamid eine Lösung der gleichen Base in 100 ml Tetrahydrofuran verwendet wird. Man erhält 38 g (80% der Theorie) 3-(2,2-Dichlorvinyl)-2,2-dimethylcyclopropan-1-carbonsäureätylester in Form eines farblosen Öls vom Siedepunkt 80-95 °C/2 mbar.

*Beispiel 4*

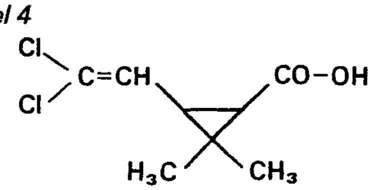

Eine Lösung von 15 g Natriumäthylat in 50 ml Dimethylformamid wird unter Rühren zu einer auf −20 °C abgekühlten Lösung von 24 g (0,1 mol) 3-Formyl-2,2-dimethylcyclopropan-1-carbonsäure und 22 g (0,1 mol) Dichlormethanphosphonsäuredieäthylester in 100 ml Tetrahydrofuran tropfenweise gegeben. Das Reaktionsgemisch wird 15 h bei Raumtemperatur (15 bis 25 °C) gerührt, unter verminderten Druck eingeengt und in 200 ml Wasser gelöst. Die wässerige Lösung wird mit Diäthyläther geschüttelt, mit Aktivkohle geklärt, nach Filtration mit Salzsäure angesäuert und dann mit Methylenchlorid geschüttelt. Die Methylenchloridphase wird mit Natriumsulfat getrocknet und filtriert; vom Filtrat wird dann das Lösungsmittel unter vermindertem Druck sorgfältig abdestilliert. Man erhält als Rückstand, welcher allmählich durchkristallisiert und bei 80 bis 85 °C schmilzt, 12 g (58% der Theorie) 3-(2,2-Dichlorvinyl)-2,2-dimethylcyclopropan-1-carbonsäure.

Als Ausgangsstoffe zu verwendende Dichlormethanphosphonsäureester der Formel III können beispielsweise wie folgt hergestellt werden:

a) 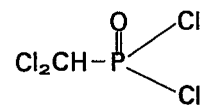

In eine Mischung aus 720 g (6 mol) Chloroform und 274 g (2 mol) Phosphortrichlorid werden portionsweise 266 g (2 mol) Aluminiumchlorid gegeben.

Das Reaktionsgemisch wird dann 12 h unter Rückfluss zum Sieden erhitzt. Anschliessend wird das Lösungsmittel im Vakuum abgezogen und der Rückstand wird in 2 l Methylenchlorid aufgenommen. Dazu werden bei 0 °C 440 ml konz. Salzsäure getropft und es wird etwa 2 h bei dieser Temperatur nachgerührt. Die organische Phase wird abgetrennt, über Natriumsulfat getrocknet und eingeengt. Der Rückstand wird fraktioniert destilliert. Man erhält 258 g (64,5% der Theorie) Dichlormethanphosphonsäuredichlorid in Form eines farblosen Öls vom Siedepunkt 48-50 °C/1 mbar.

b) 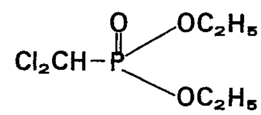

Zu einer Lösung von 227,5 g (1,13 mol) Dichlormethanphosphonsäuredichlorid in 800 ml Toluol werden bei 5-10 °C 153,5 g (2,26 mol) Natriumäthylat in 800 ml Äthanol getropft. Das Reaktionsgemisch wird 5 h bei 20 °C nachgerührt. Dann wird abgesaugt, das Filtrat eingeengt und der Rückstand fraktioniert destilliert.

Man erhält 195 g (78% der Theorie) Dichlormethanphosphonsäurediäthylester in Form eines farblosen Öls vom Siedepunkt 84 °C/1 mbar.

## Patentanspruch

Verfahren zur Herstellung von 3-(2,2-Dichlorvinyl)-2,2-dimethylcyclopropan-1-carbonsäurederivaten der Formel (I)

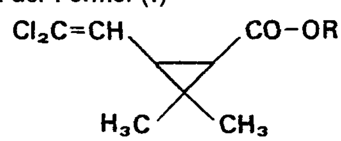 (I)

in welcher

R für Wasserstoff, ein Alkalimetall- oder Erdalkalimetalläquivalent, Alkyl oder für einen im Alkoholteil von Pyrethroiden üblichen Rest steht, dadurch gekennzeichnet, dass man 3-Formyl-2,2-dimethylcyclopropan-1-carbonsäurederivate der Formel (II)

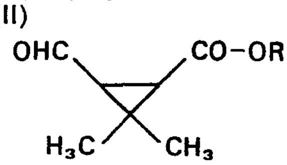 (II)

in welcher

R die oben angegebene Bedeutung hat,

mit Dichlormethanderivaten der Formel (III)

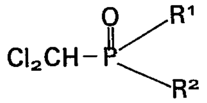

(III)

in welcher

$R^1$ und $R^2$ einzeln für Alkyl, Phenyl, Alkoxy, Phenoxy, oder zusammen für Alkandiyl (Alkylen) oder Alkandioxy (Alkylendioxy) stehen,

gegenbenenfalls unter Verwendung eines Verdünnungsmittels vorlegt und mit einem Alkoholat bei Temperaturen zwischen −50 und +50 °C versetzt und anschliessend in üblicher Weise aufarbeitet.

## Revendication

Procédé de fabrication de dérivés d'acide 3-(2,2,-dichlorovinyl)-2,2-diméthylcyclopropane-1-carboxylique de formule (I):

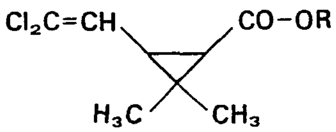

(I)

dans laquelle

R représente de l'hydrogène, un équivalent de métal alcalin ou de métal alcalino-terreux, un alcoyle ou un radical usuel dans la portion alcoolique des pyréthroïdes,

caractérisé en ce qu'on introduit d'avance des dérivés d'acide 3-formyl-2,2-diméthylcyclopropane-1-carboxylique de formule (II)

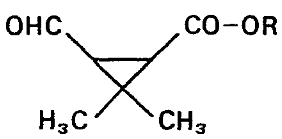

(II)

dans laquelle

R a la signification indiquée,

avec des dérivés de dichlorométhane de formule (III)

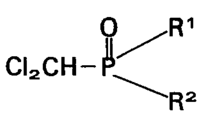

(III)

dans laquelle

$R^1$ et $R^2$ représentent individuellement un alcoyle, phényle, alcoxy, phénoxy, ou ensemble un alcanediyle (alcoylène) ou alcanedioxy (alcoylènedioxy),

éventuellement avec utilisation d'un diluant, et en ce que l'on y ajoute un alcoolate à des températures entre −50 et +50 °C, et en ce que l'on traite ensuite de la manière usuelle.

## Claim

Process for the preparation of 3-(2,2-dichlorovinyl)-2,2-dimethylcyclopropane-1-carboxylic acid derivatives of the formula (I)

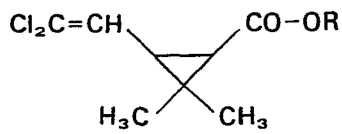

(I)

in which

R represents hydrogen, an equivalent of an alkali metal or alkaline earth metal or alkyl or a radical customary in the alcohol part of pyrethroids, characterized in that 3-formyl-2,2-dimethylcyclopropane-1-carboxylic acid derivatives of the formula (II)

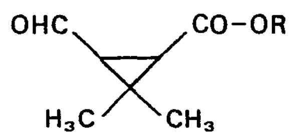

(II)

in which

R has the above-mentioned meaning,

are initially introduced together with dichloromethane derivatives of the formula (III)

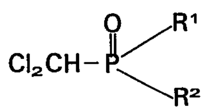

(III)

in which

$R^1$ and $R^2$ individually represent alkyl, phenyl, alkoxy or phenoxy or together represent alkanediyl (alkylene) or alkanedioxy (alkylenedioxy),

if appropriate using of a diluent and an alcoholate is added at temperatures between −50 and +50 °C and the reaction mixture is then worked up in a customary manner.